# EUROPEAN PATENT SPECIFICATION

(11) **EP 3 815 697 B1**
(45) Date of publication and mention of the grant of the patent: **03.01.2024**
(21) Application number: 19826295.8
(22) Date of filing: 10.05.2019
(51) Int. Cl.: A61L 24/08

(54) **TWO-COMPONENT HEMOSTATIC COMPOSITION AND METHOD OF MANUFACTURING SAME**
ZWEIKOMPONENTIGE HÄMOSTATISCHE ZUSAMMENSETZUNG UND VERFAHREN ZU IHRER HERSTELLUNG
COMPOSITION HÉMOSTATIQUE À DEUX CONSTITUANTS ET SON PROCÉDÉ DE PRÉPARATION

(30) Priority: 28.06.2018 KR 20180074561
(43) Date of publication of application: 05.05.2021
(73) Proprietor: Hyundai Bioland Co., Ltd., Chungcheongbuk-do 28162 (KR)
(72) Inventor: KIM, Da Yeon, Hwaseong-si Gyeonggi-do 18412 (KR); HAN, Min Eui, Cheongju-si, Chungcheongbuk-do 28123 (KR); PARK, Ki Su, Cheongju-si Chungcheongbuk-do 28115 (KR); JOO, Ji Hoon, Chungju-si Chungcheongbuk-do 28165 (KR); YANG, Eun Kyung, Cheonan-si Chungcheongnam-do 31168 (KR)
(74) Representative: J A Kemp LLP
(86) International application number: PCT/KR2019/006003
(87) International publication number: WO 2020/004813

(56) References cited:
- EP-A1- 1 849 486
- CN-A- 106 832 060
- JP-A- 2005 536 496
- KR-A- 20140 107 429
- KR-A- 20170 056 423
- KR-B1- 101 783 308
- WU, YU: "A soft tissue adhesive based on aldehyde-sodium alginate and amino-carboxymethyl chitosan preparation through the Schiff reaction", Frontiers of Materials Science, 2017, pages 215-222, XP036491190, DOI: 10.1007/s11706-017-0392-x

## Description

### Technical Field

The present invention relates to a two-liquid type hemostatic composition that may be used adjunctively during a wide range of bleeding events and surgeries, and a method for preparing the same. The two-liquid type hemostatic composition according to the present invention does not contain blood products, comprises a first solution containing chitosan and a second solution containing a glucose or cellulose compound, may be easily applied in a liquid form to a wound or surgical bleeding site of the human body, and may help hemostasis and recovery by immediately forming a hydrogel in-situ at the bleeding area.

### Background Art

In general, bleeding occurs in animals including humans when blood vessels are injured by surgical operations and physical traumas. Minor bleeding may be stopped by physiological blood coagulation without external artificial assistance, but may lead to blood loss to a degree that can have a fatal effect on body functions depending on the degree of damage. Excessive bleeding accompanied by such severe tissue damage is one of the important causes that endanger life in an instant, leading to death. It has been reported in medical circles that 74% of the causes of death of multiple injury patients among patients who visit emergency rooms are hypovolemic shock caused by excessive bleeding.

The patient's survivability may be increased by minimizing blood loss through early effective hemostasis and quickly ensuring a surgical field of view to shorten the operation time. Thus, the use of hemostatic agents in operating rooms, general emergency situations, and battlefields is essential.

Conventional hemostatic agents may be broadly classified into three types: a glue type that may be applied in a liquid form; a patch or sponge type having a mesh structure that absorbs blood and presses a wound; and a powder type that is applied to a wound. The liquid-type hemostatic agent is applicable to various wounds, but has disadvantages in that it requires time for thawing, mixing and the like and has a low hemostatic effect. The patch-type hemostatic agent does not require any preparation and is easy to store, but requires additional pressure for the application thereof, and thus the applicability thereof to deep wounds is low. The powder-type hemostatic agent is applicable to irregular bleeding sites, but has disadvantages in that it is difficult to apply uniformly to the wound surface and difficult to remove after application.

In addition, hemostatic agents containing blood products such as thrombin and fibrinogen and hemostatic agents containing collagen are expensive, and their usability and storage stabilities are poor. Furthermore, these hemostatic agents also have a problem of hypersensitivity and the risk of infection by viral pathogens such as HIV, HBV, HCV and CMV based on blood products.

The liquid-type hemostatic agent Floseal (Baxter), which is currently commercially available, is inconvenient to use because it is required to prepare a thrombin solution by mixing dry thrombin with a calcium chloride solution at a surgical site and finally mix the thrombin solution with a gelatin matrix. In addition, the liquid-type hemostatic agent Floseal has a time constraint in that it should be used within 2 hours after mixing.

These liquid-type hemostatic agents based on blood products are used limitedly for small affected areas due to the high prices thereof, have low storage stability, and cause a risk of infection with certain diseases. In addition, since hemostasis occurs based on the principle of activating blood coagulation, these liquid-type hemostatic agents have a limitation in that they do not work on hemophilia patients and diabetic patients who have impairment in the blood coagulation system, and patients who take anticoagulants and aspirin.

Meanwhile, chitosan, a representative cationic polysaccharide, functions to induce quick blood coagulation, and thus was officially approved for use as a bandage additive or a hemostatic agent in the United States and Europe. A hemostatic product made of chitosan proved its performance in an experiment on rapid hemostasis and blood loss prevention, which was conducted by the US Marine Corps. In addition, the hemostatic product has very little irritation and antibacterial properties, and thus is widely used for medical purposes. However, it is difficult to expect excellent hemostatic efficacy by chitosan alone, and chitosan has a problem in that it has low solubility in a neutral solution and dissolves in an acidic solution. Due to this problem, chitosan causes toxicity when used for medical purposes, and thus there are many restrictions on the practical use of chitosan. A two-liquid type hemostatic composition comprising chitosan and oxidized dextran is disclosed in Patent Document 3.

Therefore, there is still a need to develop an improved hemostatic agent having excellent blood coagulation properties and hemostatic performance compared to the hemostatic agents developed in the prior art.

Accordingly, the present inventors have made extensive efforts to overcome the above-described problems occurring in the prior art, and as a result, have found that a two-liquid type hemostatic composition as claimed may be easily applied in a liquid form to a wound or surgical bleeding site of the human body, and may exhibit excellent blood coagulation properties and hemostatic performance by immediately forming a hydrogel at a bleeding site even without containing a blood product, thereby completing the present invention.

[Prior Art Documents]
(Patent Document 1) KR 10-1429455 B
(Patent Document 2) KR 10-1829136 B
(Patent Document 3) EP 1 849 486 A1

### DISCLOSURE

### Technical Problem

Therefore, a technical problem to be solved by the present invention relates to a hemostatic composition not containing a blood product (thrombin or fibrinogen), and is to provide a liquid hemostatic agent, which is based on a hemostasis-induced functional biomaterial and may be easily applied to a bleeding site, and a method for preparing the same.

### Technical Solution

In accordance with one aspect of the present invention, the present invention provides a two-liquid type composition for use as a blood coagulating agent, said composition comprising: a first solution containing the first component which is a chitosan compound having a cationic amino acid conjugated to a side chain thereof; and a second solution containing the second component which is selected from dextran, hyaluronic acid, carboxymethylcellulose or alginate compound having an aldehyde group introduced therein by oxidation, wherein, when the first solution and the second solution are mixed together, a hydrogel is formed by a Schiff base reaction between the amine (-NH₂) functional group of the chitosan compound and the aldehyde (-CHO) functional group of the second component simultaneously with the electrostatic interaction between opposite charges of the chitosan compound and the second component.

In the present specification, the term "two-liquid type" means that the composition is composed of two liquids: the first solution, and the second solution. The two liquids (solutions) have fluidity, and thus are easily mixed together. In addition, the term "hemostatic agent or composition" refers to a substance or a composition capable of inducing clotting or plugging to stop or reduce bleeding when coming into contact with blood or other body fluids. In addition, the term "hydrogel" refers to a network structure in which a water-soluble polymer forms a three-dimensional crosslink by a physical bond (hydrogen bond, etc.) or a chemical bond (covalent bond, etc.). When hydrogel is mixed with blood, it may induce blood coagulation, and may also physically press a bleeding site.

In the present specification, the chitosan compound, contained in the first solution and having a cationic amino acid conjugated to a side chain thereof, is referred to as a first component, and the dextran, hyaluronic acid, carboxymethylcellulose or alginate compound, contained in the second solution and having an aldehyde group introduced therein by oxidation, is referred to as a second component.

The present invention provides a two-liquid type hemostatic composition wherein, when the first solution and the second solution are applied to a bleeding site, the following steps occur simultaneously: a step of inducing blood coagulation by the electrostatic interaction between opposite charges of material and material (the cationic first component of the first solution and the anionic second component of the second solution) and between opposite charges of material and blood (the cationic first component of the first solution and the anionic red blood cells of blood); a step of inducing blood coagulation by a Schiff base reaction between material and material (the amine functional group of the first component of the first solution and the aldehyde functional group of the second component of the second solution) and between material and blood (the aldehyde functional group of the second component of the second solution and the platelet and protein of blood); and a step of stopping bleeding by physically pressing a bleeding site by a hydrogel formed through sol-gel transition that occurs through multiple reactions. The two-liquid type hemostatic composition of the present invention may induce more effective hemostasis than a single-component hemostatic agent due to such multifunctional effects.

The present invention provides a two-liquid type hemostatic composition wherein the chitosan compound of the first solution independently has a cationic property, is a chitosan compound in which one or more cationic amino acids selected from among arginine, histidine, cysteine and lysine, which contain one carboxyl group and one or more amino groups, are conjugated to the side chain of chitosan, and the chitosan compound dissolves at neutral pH. Chitosan before modification dissolves only at acidic pH, whereas the chitosan compound having a cationic amino acid conjugated to a side chain thereof according to the present invention may easily dissolve even at neutral pH, and may undergo a Schiff base reaction with an aldehyde group due to the amino group thereof.

The present invention provides a two-liquid type hemostatic composition wherein the second component of the second solution independently has a neutral or anionic property, and is one or more compounds selected from among a compound having an aldehyde group introduced therein by oxidation of dextran, hyaluronic acid, carboxymethyl cellulose or alginate.

In accordance with another aspect of the present invention, the present invention provides a method for preparing the two-liquid type hemostatic composition, the method comprising steps of: preparing a first solution by dissolving, in distilled water or physiological saline (e.g., PBS, etc.), the first component which is a chitosan compound having a cationic amino acid conjugated to a side chain thereof; and preparing a second solution by dissolving, in distilled water or physiological saline, the second component which is selected from dextran, hyaluronic acid, carboxymethylcellulose or alginate compound having an aldehyde group introduced therein by oxidation.

In the present invention, the first solution may contain the chitosan compound, having a cationic amino acid conjugated to a side chain thereof, at a concentration of 0.5 to 5 wt%, and the second solution may contain a second compound which is selected from dextran, hyaluronic acid, carboxymethylcellulose or alginate compound having an aldehyde group introduced therein by oxidation, at a concentration of 1 to 15 wt%. In addition, the first solution and the second solution may be mixed together at a volume ratio of 1:12 to 12:1 to form a hydrogel.

The present invention provides a two-liquid type hemostatic composition wherein the first solution and the second solution are stored in separate chambers, and when the first solution and the second solution are applied to a bleeding site, they are mixed together, thus immediately forming a hydrogel in-situ at the bleeding site.

The present invention provides a two-liquid type hemostatic composition wherein the first solution and the second solution are contained in a dual chamber syringe, and a method of applying them is selected by assembling one of a needle, a mixing nozzle, a spreader and a spray-type type auxiliary tool to the inlet of the dual chamber syringe.

The present invention provides a two-liquid type hemostatic composition wherein any one or both of the first solution and the second solution further contain cells, a drug or an anti-adhesion material.

The present invention provides a two-liquid type hemostatic composition wherein the two-liquid type hemostatic composition comprises cells and functions as a tissue engineering scaffold by the formed hydrogel. The cells may be any type of cells, such as embryonic stem cells or adult stem cells, which may be contained in the tissue engineering scaffold, and the hydrogel may function as a scaffold in which the cells may be cultured to differentiate into tissue.

The present invention provides a two-liquid type hemostatic composition wherein the two-liquid type hemostatic composition comprises a drug and functions as a drug carrier by the formed hydrogel. The drug may be any drug intended for administration to the human body, and the hydrogel may function as a drug carrier by slowly releasing the drug contained therein.

The present invention provides a two-liquid type hemostatic composition wherein the two-liquid type hemostatic composition comprises an anti-adhesion material and functions as an anti-adhesion agent by the formed hydrogel. The anti-adhesion material may be any anti-adhesion material that has been used in a conventional art, a hydrogel itself may act as the anti-adhesion material, and the hydrogel may function as an anti-adhesion agent by preventing adhesion between tissues after surgery.

Hereinafter, the present invention will be described in detail.

The composition of the present invention is defined by claim 1.

In the present invention, chitosan, a polysaccharide, was used as the first component to induce the known blood coagulation function of the chitosan, and furthermore, a cationic amino acid compound was introduced to a side chain of chitosan. The chitosan having a cationic amino acid introduced thereto may dissolve at neutral pH, and thus poses no toxicity problem when applied *in vivo,* and it is a cationic biocompatible polymer that may induce blood coagulation by electrostatic interaction with anionic red blood cells and platelets.

As the second component, a material having an aldehyde (-CHO) functional group was produced by oxidation of dextran, hyaluronic acid, carboxymethylcellulose or alginate. This material may undergo a Schiff base reaction immediately upon reaction with the amine (-NH₂) functional group of the chitosan compound as the first component, thus forming a hydrogel. When a liquid hemostatic agent is applied to a bleeding site, the blood and the material are mixed together to form a gel, so that the blood coagulates immediately, and furthermore, the formed gel may exhibit a hemostatic effect by physically pressing the bleeding site.

The present invention provides a hydrogel-type hemostatic composition comprising: (A) a first compound as a chitosan compound in which an amino acid containing one carboxyl (-COOH) group and one or more amino (-NH₂) groups is conjugated to the side chain of chitosan; and (B) a second compound as an oxidized dextran, hyaluronic acid, carboxymethylcellulose or alginate compound containing an aldehyde group, and a method for preparing the composition.

In order to achieve the purpose of hemostasis, the hydrogel-type hemostatic composition comprises steps of: a) synthesizing compounds as first and second components based on the Examples described below; b) selecting two compounds from among the synthesized compounds, and preparing two solutions containing the selected compounds, respectively, while adjusting the concentrations of the compounds according to a bleeding site and the need of the user; c) applying the two solutions to an application tool including a syringe; and d) applying the solutions to the bleeding site to induce hemostasis.

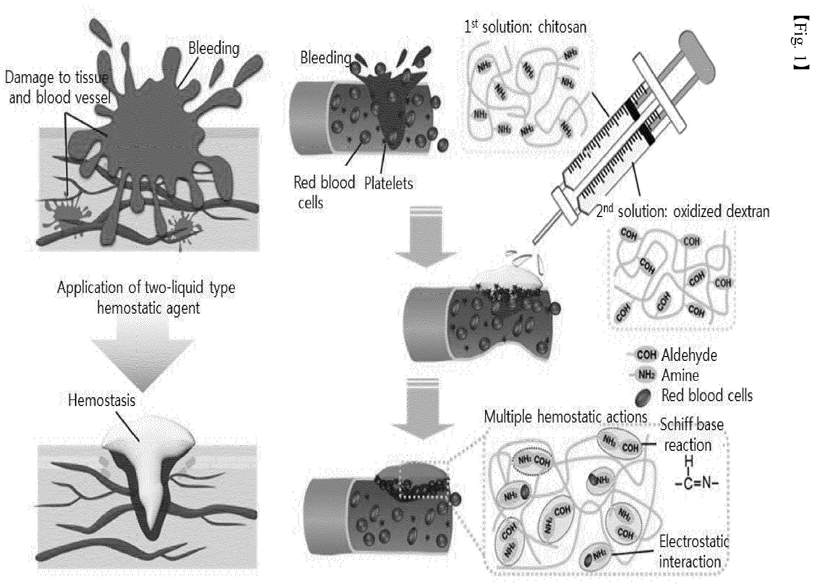

In step c), the mixing volume ratio between the two solutions may be adjusted to a ratio of 1:12 to 12:1, and in step d), the two solutions are preferably applied simultaneously to the bleeding site by means of a dual syringe (or a double or twin syringe) as schematically shown in FIG. 1.

At this time, as the solutions are mixed with the blood, they may exhibit immediate blood coagulation and hemostatic effects. Here, blood coagulation and hemostasis are characterized in that the following steps occur simultaneously: a step of inducing blood coagulation by the electrostatic attraction between opposite charges of material and material and material and blood; a step of inducing blood coagulation by a Schiff base reaction between material and material and between material and blood; and a step of stopping bleeding by physically pressing a bleeding site by a hydrogel formed through sol-gel transition that occurs through multiple reactions. The composition of the present invention may induce more effective hemostasis than a single-component hemostatic agent due to such multifunctional effects.

Chitosan which is the main component of the first component in the present invention is a material obtained by deacetylation of chitin isolated from crustaceans such as crab, crayfish, and shrimp, is composed of D-glucosamine and N-acetyl-glucosamine monomers, and generally has a degree of deacetylation of 60 to 97%. Since chitin has a strong and solid molecular bond and is insoluble in water and organic solvents, solubility is imparted thereto by a deacetylation process, but chitosan still has the disadvantage of dissolving in acidic solutions.

Chitosan is known to show a difference in solubility and *in-vivo* efficacy because the content of amine groups contained in glucosamine varies depending on the degree of deacetylation. In addition, chitosan has a structural advantage in that functional groups capable of imparting various functions may be conjugated to a large amount of amino groups in the side chain thereof.

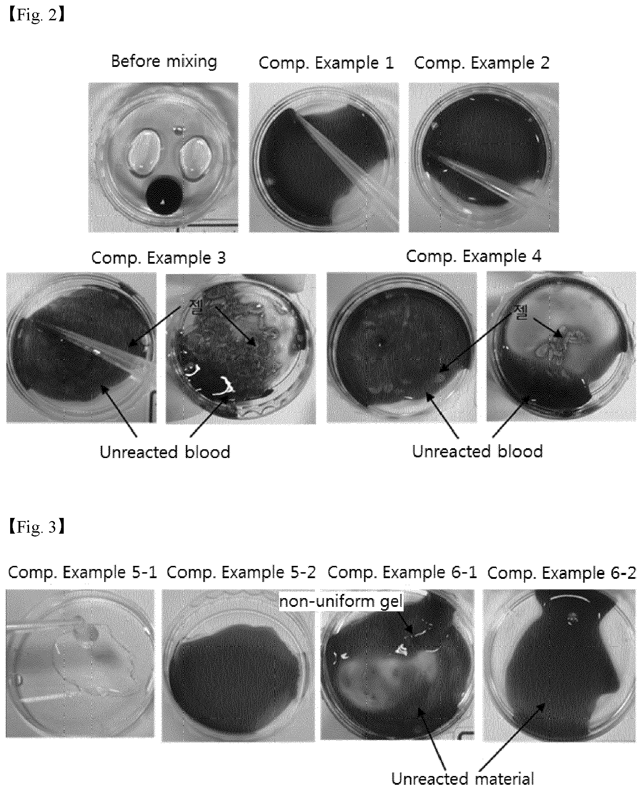

Although chitosan is used as a representative cationic natural biopolymer, the application of chitosan as a hemostatic agent in studies prior to the present invention was limited due to the problem that chitosan dissolves at acidic pH. For example, a solution of chitosan in a 1% HCl aqueous solution and a solution of a neutral glucose or cellulose compound, which are composed of opposite charges, induced blood coagulation, but the chitosan material precipitated immediately after mixing of the solutions, or blood coagulation having desired performance did not occur (Comparative Examples 1 to 4 in FIG. 2).

In addition, studies have been conducted to solve the solubility problem of chitosan by conjugating particularly functional groups to the amino group in the side chain of chitosan. For example, based on the literature of Tan H. et al. (Biomaterials. 2009; 30(13):2499-506), succinyl modified chitosan was produced and mixed with an oxidized glucose or cellulose compound, but was not suitable as a hemostatic agent because non-uniform gel and unreacted material were observed (Comparative Example 5-1 in FIG. 3: blood coagulation action of a combination of 2.5% succinyl modified chitosan and 10% CMC-Al, and Comparative Example 5-2: blood coagulation action of a combination of 2.5% succinyl modified chitosan and 10% DEX-Al). For another example, based on the literature of Amidi M. et al. (J Control Release. 2006; 111(1-2):107-16), trimethyl chitosan was produced and mixed with an oxidized glucose or cellulose compound, but was not suitable as a hemostatic agent because non-uniform gel and unreacted material were observed (Comparative Example 6-1 in FIG. 3: blood coagulation action of a combination of 2.5% trimethyl chitosan and 1% HA-Al; Comparative Example 6-2: blood coagulation action of a combination of 2.5% trimethyl chitosan and 10% CMC-Al). In the results of the previous studies, after a functional group was introduced to the amino group of the side chain of chitosan, a problem arose in that the cationic property of the chitosan was lost and the inherent performance of the chitosan was degraded. Due to this problem, desired blood coagulation was not induced, even though the chitosan partially formed a gel.

Therefore, a compound conjugated to the side chain of chitosan for use as a hydrogel-type hemostatic agent of the present invention is designed such that the cationic property of chitosan is retained and chitosan has the property of dissolving at neutral pH by conjugation of the compound. One or more of arginine, histidine, cysteine and lysine, which are representative cationic amino acids that easily dissolve in water, may be conjugated to the side chain of chitosan, and one or more amino acids may be repeatedly conjugated by an amide bond.

The amino acid conjugated to the side chain of chitosan for use as a hydrogel-type hemostatic agent of the present invention is preferably lysine, but is not limited thereto and may also be selected from among cysteine, arginine and histidine depending on the hemostatic site performance and properties.

The following Formula 1 represents a first component as a chitosan compound having lysine introduced to the side chain of chitosan:

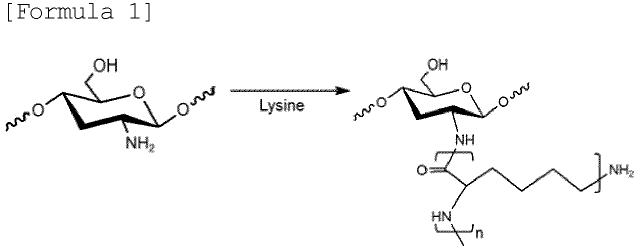

In Formula 1 above, n is an integer ranging from 0 to 20, preferably an integer ranging from 1 to 3, which indicates the number of repeating units of lysine introduced to the side chain of chitosan.

The production of the chitosan compound as the first compound comprises a process of treatment with a crosslinker to the above-specified amino acid to the side chain of chitosan. At this time, the chitosan compound as a cationic biopolymer may be produced by reaction between the amino group of the side chain of chitosan and the carboxyl group of the cationic amino acid compound, and the crosslinker may be one or more selected from among a water-soluble carbodiimide compound, an organic solvent-soluble carbodiimide compound, an aldehyde compound, and an isocyanate compound. More preferably, examples of the crosslinker include, but are not limited to, 1-(3-dimethylaminopropyl)-3-ethylcarbodiimide hydrochloride (EDAC or EDC), N,N'-dicyclohexylcarbodiimide (DCC), and N-hydroxysuccinimide (NHS).

The second component of the present invention is produced from dextran, hyaluronic acid (HA), carboxymethyl cellulose (CMC), or alginate, and comprises one or more of oxidized dextran, oxidized hyaluronic acid, oxidized carboxymethyl cellulose, and oxidized alginate, which have an aldehyde (-CHO) group introduced therein by oxidation with periodate as an oxidizing agent.

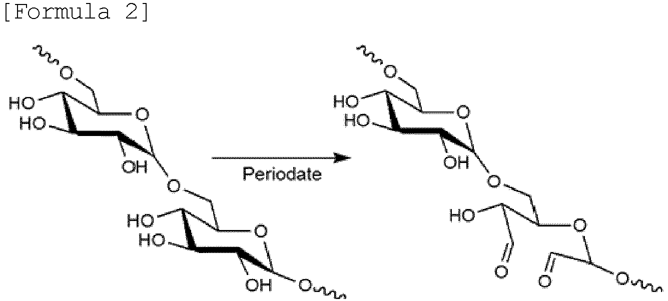

Formula 2 above represents oxidized dextran as the second component compound. Depending on the type of the first component, the second component may be selected from among oxidized dextran, oxidized hyaluronic acid, oxidized carboxymethyl cellulose, oxidized alginate, and oxidized starch. Among the second component compounds, dextran oxide is particularly preferred for use as a hemostatic agent in terms of the rate of reaction with the first component chitosan compound, the physical properties of the hydrogel, the uniformity of mixing with blood, and the like. The average molecular weights of the first component and the second component may have a specific average molecular weight distribution selected depending on the specific required performance of a bleeding site to which they are applied and the degradation period. It was confirmed through the Examples of the present invention that when the molecular weight was high, the degradation period could be prolonged, whereas, when the molecular weight was low, the reaction rate for blood coagulation could be increased.

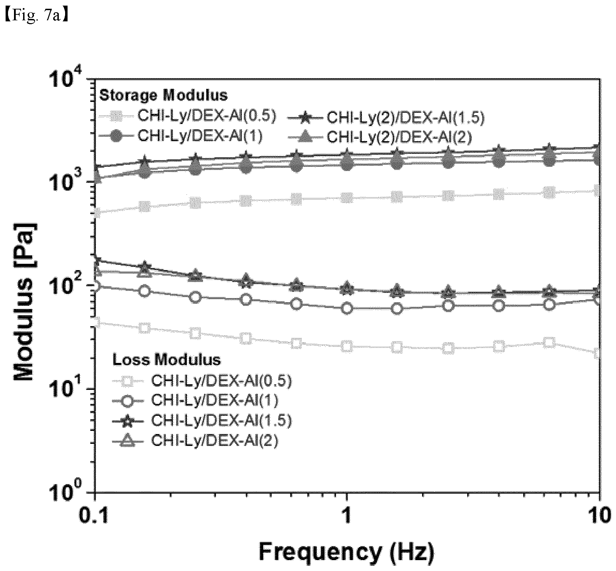

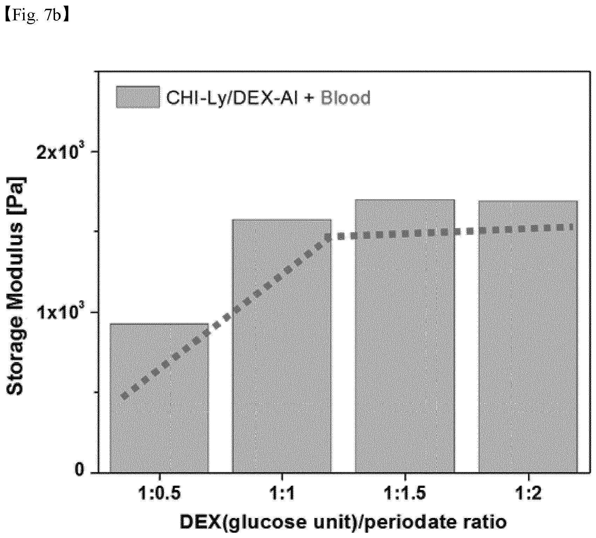

It is known that the oxidation reactivity of the second component is influenced by temperature, pH and the concentration of the oxidizing agent. The present invention provides a method of introducing an aldehyde group by oxidation with periodate. As a result of evaluating the hemostatic performance of oxidized dextran produced in Production Example 4 of the present invention, it was confirmed that, when the amount of periodate used was 1.5 equivalents or more, the oxidation degree and physical properties did not increase and converged. Therefore, it is preferable to treat glucose with 1.5 equivalents or more of periodate in order to induce a certain level or higher of physical properties. The results are shown in FIG. 7.

### Advantageous Effects

According to the present invention as described above, the hydrogel-type hemostatic agent is a hemostatic agent that does not contain blood products such as thrombin and fibrinogen, and may be applied to hemophilia patients and diabetic patients who have impairment in the blood coagulation system, and patients taking an anticoagulant and aspirin, in order to induce blood coagulation and hemostasis. In addition, it may be used as a hemostatic agent having a more effective hemostatic effect in a wide range of surgical operation areas because multiple reactions occur simultaneously.

### Brief Description of Drawings

FIG. 1 is schematic representation showing inducing blood coagulation and hemostasis by applying a hydrogel-type hemostatic agent of the present invention to a bleeding site.
FIG. 2 shows the results of evaluating the blood coagulation action of a combination of a pre-modified chitosan solution and a glucose or cellulose compound solution, and specifically depicts images of Comparative Examples 1 to 4.
FIG. 3 shows the results of evaluating the blood coagulation action of a combination of a chitosan solution and a glucose or cellulose compound solution, which has introduced therein a functional group different from that used in the present invention, and specifically depicts images of Comparative Example 5-1 through Comparative Example 6-2.
FIG. 4 shows the results of evaluating the blood coagulation action of a combination of the first solution and the second solution of the present invention, and specifically depicts images of Examples 3-1 to 3-14.
FIG. 5 shows the results of evaluating the blood coagulation action of a combination of the first solution and the second solution of the present invention, and specifically depicts images of Examples 3-15 to 3-21.
FIG. 6 shows the results of evaluating the blood coagulation action of a combination of the first solution and the second solution of the present invention, and specifically depicts images of Examples 3-22 to 3-26.
FIG. 7 depicts a graph (FIG. 7(A)) showing the results of measuring rheometer properties as a function of the ratio of an oxidizing agent per glucose repeating unit, and graph comparing storage modulus (FIG. 8(B)) at frequency 1.
FIG. 8 depicts a graph (FIG. 8(A)) showing the results of measuring rheometer properties as a function of the molecular weight of oxidized dextran, and graphs comparing storage modulus (FIG. 8(B)) and viscosity (FIG. 8(C)) at frequency 1.
FIG. 9 shows images of blood coagulation evaluation of the liquid hemostatic agent Tisseel (FIG. 9(A)) and images obtained using a dual chamber syringe and an application cannula (FIG. 9(B)) .
FIG. 10 depicts a graph showing the results of measuring the rheometer properties of the liquid hemostatic agent Tisseel and Example 3-1 (FIG. 10(A)), and graphs comparing storage modulus (FIG. 10(B)) and viscosity (FIG. 10(C)) at frequency 1.
FIG. 11 depicts images of first and second solutions as hemostatic solutions and the application of the solutions to a dual chamber syringe.

### Mode for Invention

Hereinafter, the present invention will be described in more detail with reference to examples. These examples are only to illustrate the present invention.

### <Production Example 1> Production of Lysine-Conjugated Chitosan (CHI-Ly)

This Production Example was intended to produce first components using chitosan raw materials having different average molecular weights and deacetylation degrees, and detailed reaction raw material specifications and reaction conditions are shown in Table 1 below. First, a chitosan solution was prepared by sufficiently dissolving 1 g of chitosan in 150 ml of 1%(v/v) HCl aqueous solution. The chitosan solution was adjusted to pH 5 by adding 1 M of NaOH aqueous solution thereto dropwise. According to the molar ratio per repeating unit shown in Table 1 below, 1-(3-dimethylaminopropyl)-3-ethylcarbodiimide hydrochloride (EDAC) was dissolved in distilled water and added slowly to the chitosan solution. After titration from pH 4 to 5 with 1 M of HCl aqueous solution, lysine dissolved in HCl aqueous solution was added slowly to the chitosan solution. Then, the pH was checked and titrated from pH 4 to 5, a reaction was performed by stirring the reaction solution at room temperature for 6 hours. After the reaction, the obtained compound was subjected to dialysis for 72 hours or more to remove reaction by-products and unreacted materials. Then, the compound was cooled to a temperature of -80°C and freeze-dried, thereby producing lysine-conjugated chitosan compounds as first components.

**[Table 1]**

| No. | Chitosan average molecular weight (kDa) | Deacetylati on degree (%) | Reaction ratio* (molar ratio) repeating unit of chitosan : lysine | Lysine weight** (g) |
|---|---|---|---|---|
| 1 | 3 to 20 kDa Chito-oligosacchar ide | 70% | 1 : 0.5 | 0.294 |
| | | | 1 : 1 | 0.589 |
| | | | 1 : 2 | 1.178 |
| 2 | 50 to 190 kDa | 80% | 1 : 0.5 | 0.345 |
| | | | 1 : 1 | 0.690 |
| | | | 1 : 2 | 1.379 |
| 3 | 110 to 150 kDa | 95% | 1 : 0.5 | 0.425 |
| | | | 1 : 1 | 0.851 |
| | | | 1 : 2 | 1.701 |
| 4 | 200 kDa | 85% | 1 : 0.5 | 0.371 |
| | | | 1 : 1 | 0.742 |
| | | | 1 : 2 | 1.484 |
| 5 | 150 to 250 kDa | 95% | 1 : 0.5 | 0.425 |
| | | | 1 : 1 | 0.851 |
| | | | 1 : 2 | 1.701 |
| 6 | 190 to 310 kDa | 80% | 1 : 0.5 | 0.345 |
| | | | 1 : 1 | 0.690 |
| | | | 1 : 2 | 1.379 |
| 7 | 300 to 340 kDa | 95% | 1 : 0.5 | 0.425 |
| | | | 1 : 1 | 0.851 |
| | | | 1 : 2 | 1.701 |
| 8 | 310 to 375 kDa | 75% | 1 : 0.5 | 0.319 |
| | | | 1: 1 | 0.639 |
| | | | 1 : 2 | 1.277 |

| | | | | |
|---|---|---|---|---|
| * The reaction ratio is the reaction molar ratio of lysine per glucosamine repeating unit except N-acetylglucosamine of chitosan. ** The lysine weight is the weight of lysine added per g of chitosan, calculated by *the reaction ratio. | | | | |

### <Production Example 2> Production of Cysteine-Conjugated Chitosan (CHI-Sh)

A chitosan solution was prepared by sufficiently dissolving 1 g of chitosan in 150 ml of 1%(v/v) HCl aqueous solution. Each of 1.51, 0.76 and 0.30 g of cysteine was dissolved in 50 ml of 1%(v/v) HCl aqueous solution and added slowly to the chitosan solution which was then titrated to a pH 5 by adding 1 M of NaOH aqueous solution thereto dropwise. Each of 2.4, 1.2 and 0.5 g of EDAC was dissolved in 30 ml of distilled water and added slowly for 20 minutes. Next, the pH was checked and titrated from pH 4 to 5, and then a reaction was performed by stirring the reaction solution at room temperature for 6 hours. Each of the obtained compounds was subjected to dialysis for 72 hours or more to remove reaction by-products and unreacted materials. Then, each of the compounds was cooled to a temperature of -80°C and freeze-dried, thereby producing cysteine-conjugated chitosan compounds as first components.

### <Production Example 3> Production of Arginine-Conjugated Chitosan (CHI-Ar)

A chitosan solution was prepared by sufficiently dissolving 1 g of chitosan in 150 ml of 1%(v/v) HCl aqueous solution. Each of 1.64, 0.82 and 0.41 g of arginine was dissolved in 50 ml of 1%(v/v) HCl aqueous solution and added slowly to the chitosan solution which was then titrated to a pH 5 by adding 1 M of NaOH aqueous solution thereto dropwise. Then, each of 2.17, 1.08 and 0.54 g was dissolved in 30 ml of EDAC and added slowly to the chitosan solution for 20 minutes. Next, the pH was checked and titrated from pH 4 to 5, and then a reaction was performed by stirring the reaction solution at room temperature for 6 hours. Each of the obtained compounds was subjected to dialysis for 72 hours or more to remove reaction by-products and unreacted materials. Then, each of the compounds was cooled to a temperature of -80°C and freeze-dried, thereby producing arginine-conjugated chitosan compounds as first compounds.

### <Production Example 4> Production of Oxidized Dextran (DEX-Al)

This Production Example was intended to produce second components using dextran raw materials having different average molecular weights, and detailed reaction raw material specifications and reaction conditions are shown in Table 2 below. First, a dextran solution was prepared by sufficiently dissolving 5 g of dextran in 100 ml of distilled water. According to the reaction molar ratio per repeating unit shown in Table 2 below, sodium periodate (NaIO₄) dissolved in distilled water was added slowly to the dextran solution in dark conditions. While room temperature and dark conditions were maintained, a reaction was performed by stirring the reaction solution for 6 hours. Then, 5 ml of ethylene glycol was added to the reaction solution and stirred for 1 hour, and the reaction was terminated. After the reaction, the obtained compound was subjected to dialysis for 72 hours or more to remove reaction by-products and unreacted materials. Then, the compound was cooled to a temperature of -80°C and freeze-dried, thereby producing aldehyde group-containing oxidized dextran compounds as second components.

**[Table 2]**

| No. | Dextran average molecular weight (kDa) | Reaction ratio^{#} (molar ratio) repeating unit of dextran : oxidizing agent | Oxidizing agent^{##} (g) |
|---|---|---|---|
| 1 | 9 to 11 kDa | 1 : 0.5 | 0.39 |
| | | 1 : 1 | 0.78 |
| | | 1 : 1.5 | 1.17 |
| | | 1 : 2 | 1.56 |
| 2 | 35 to 45 kDa | 1 : 0.5 | 0.39 |
| | | 1 : 1 | 0.78 |
| | | 1 : 1.5 | 1.17 |
| | | 1 : 2 | 1.56 |
| 3 | 40 kDa | 1 : 0.5 | 0.39 |
| | | 1 : 1 | 0.78 |
| | | 1 : 1.5 | 1.17 |
| | | 1 : 2 | 1.56 |
| 4 | 70 kDa | 1 : 0.5 | 0.39 |
| | | 1 : 1 | 0.78 |
| | | 1 : 1.5 | 1.17 |
| | | 1 : 2 | 1.56 |
| 5 | 150 kDa | 1 : 0.5 | 0.39 |
| | | 1 : 1 | 0.78 |
| | | 1 : 1.5 | 1.17 |
| | | 1 : 2 | 1.56 |
| 6 | 200 kDa | 1 : 0.5 | 0.39 |
| | | 1 : 1 | 0.78 |
| | | 1 : 1.5 | 1.17 |
| | | 1 : 2 | 1.56 |

| | | | |
|---|---|---|---|
| ^{#}The reaction ratio is the reaction molar ratio of the oxidizing agent per glucose repeating unit of dextran. ^{##}The oxidizing agent weight is the weight of oxidizing agent added per g of dextran, calculated by ^{#}the reaction ratio. | | | |

### <Production Example 5> Oxidized Hyaluronic Acid (HA-Al)

A hyaluronic acid solution was prepared by stirring hyaluronic acid having an average molecular weight of each of 700 and 3,000 kDa in 500 ml of distilled water at a concentration of 1% or 0.5% for 2 days or more. 0.6 g of sodium periodate dissolved in distilled water was added slowly to the hyaluronic acid solution in dark conditions. While the room temperature and dark conditions were maintained, a reaction was performed by stirring the reaction solution for 3 hours. Then, 5 ml of ethylene glycol was added to the reaction solution which was then additionally stirred for 1 hour, and the reaction was terminated. After the reaction, the obtained compound was subjected to dialysis for 72 hours or more to remove reaction by-products and unreacted materials. Then, the compound was cooled to a temperature of -80°C and freeze-dried, thereby aldehyde group-containing oxidized hyaluronic acids as second components.

### <Production Example 6> Oxidized Carboxymethyl Cellulose (CMC-Al)

A carboxymethyl cellulose solution was prepared by sufficiently dissolving 2 g of carboxymethyl cellulose having an average molecular weight of each of 90, 250 and 700 kDa in 50 ml of distilled water. 1.1 g of sodium periodate dissolved in 10 ml of distilled water was added slowly to the carboxymethyl cellulose solution in dark conditions. While room temperature and dark conditions were maintained, a reaction was performed by stirring the reaction solution for 6 hours. Then, 5 ml of ethylene e glycol was added to the reaction solution which was then additionally stirred for 1 hour, and the reaction was terminated. After the reaction, the obtained compound was subjected to dialysis for 72 hours or more to remove reaction by-products and unreacted products. Then, the product was cooled to a temperature of -80°C and freeze-dried, thereby producing aldehyde group-containing oxidized carboxymethyl cellulose compounds as second components.

### <Example 7> Oxidized Alginate (ALG-Al)

An alginate solution was prepared by sufficiently dissolving 5 g of alginate having an average molecular weight of 120 to 190 kDa in 25 ml of ethanol. 1.6 g of sodium periodate dissolved in 25 ml of distilled water was added slowly to the alginate solution in dark conditions. While room temperature and dark conditions were maintained, a reaction was performed by stirring the reaction solution for 3 hours. Then, 5 ml of ethylene glycol was added to the reaction solution which was then additionally stirred for 1 hour, and the reaction was terminated. After the reaction, the obtained compound was subjected to dialysis for 72 hours or more to remove reaction by-products and unreacted materials. Then, the product was cooled to a temperature of -80°C and freeze-dried, thereby aldehyde group-containing oxidized alginate compounds as second components.

### <Example 1> Preparation of Hemostatic Solution

Each of the compounds produced by the above production method may be prepared in the form of sponge or powder. Hemostatic solutions (first solution and second solution) were prepared by dissolving each of the first component and second component compounds in distilled water or PBS. 1.25% and 2.5% chitosan-lysine (CHI-Ly) solution, 1.25% and 2.5% chitosan-cysteine (CHI-Sh) solution, 2.5% chitosan-arginine (CHI-Ar) solution, 8%, 9% and 20% oxidized dextran (DEX-Al) solution, 2% oxidized hyaluronic acid (HA-Al) solution, 10% oxidized carboxymethyl cellulose (CMC-Al) solution, and 10% oxidized alginate (ALG-Al) solution were prepared, stabilized at 4°C for 24 hours, and then used for evaluation. The concentrations of the first component and the second component solution may be adjusted according to the needs of the user in consideration of the molecular weight and the viscosity of the solution.

### <Example 2> Measurement and Evaluation of Zeta Potential

Solutions for measurement were prepared by each of the hemostatic solutions (CHI-Ly, CHI-Sh, DEX-Al, HA-Al, CMC-Al, and ALG-Al), prepared in Example 1, 50-fold with distilled water. For comparison with the hemostatic solutions, 50-fold dilutions of 2.5% chitosan solution (CHI) and 8% dextran solution (DEX) were prepared. At this time, the chitosan solution was dissolved in 1%(v/v) HCl aqueous solution, and then diluted 50-fold in distilled water. The Zeta potential of each of the compounds was measured using an ELSZ-1000 (Otsuka Electronics; Japan) device, and the results of the measurement are shown in Table 3 below.

As shown in Table 3 below, CHI, CHI-Ly and CHI-Sh showed positive charge properties, and HA-Al, CMC-Al, ALG-Al, DEX and DEX-Al showed negative charge properties. These results indicate that the first component and second component solutions have opposite charges, and thus may form a gel by electrostatic interaction. In addition, CHI-Ly and DEX-Al were mixed together, and the Zeta potential of the mixture was measured. As a result, the mixture showed a property of positive charge (37.2 ± 1.1), suggesting that the two-liquid type hemostatic agent is advantageous for blood coagulation by electrostatic interaction with blood components.

**[Table 3]**

| Hemostatic biomaterial | | No. | | | Average | Remarks |
|---|---|---|---|---|---|---|
| | | 1 | 2 | 3 | | |
| 1 | Chitosan (CHI) | 54.4 | 56.0 | 56.5 | 55.6 ± 1.1 | Positive charge |
| 2 | Dextran (DEX) | - 5.0 | -7.1 | -8.1 | -6.7 ± 1.6 | Negative charge |
| 3 | Chitosan-lysine (CHI-Ly) | 47.7 | 46.8 | 49.0 | 47.8 ± 1.1 | Positive charge |
| 4 | Oxidized dextran (DEX-Al) | -3.6 | -4.4 | -2.6 | -3.5 ± 0.9 | Negative charge |
| 5 | Oxidized hyaluronic acid (HA-Al) | -54.5 | -53.2 | -52.4 | -53.4 ± 1.1 | Negative charge |
| 6 | Oxidized carboxymethyl cellulose (CMC-Al) | -43.8 | -44.3 | -46.8 | -45.0 ± 1.6 | Negative charge |
| 7 | Oxidized alginate (ALG-Al) | -53.7 | -53.3 | -56.3 | -54.5 ± 1.6 | Negative charge |

### <Example 3> Evaluation of Blood Coagulation Action

Blood coagulation evaluation of the hemostatic solutions prepared in Example 1 was performed, and as a result, it was confirmed that the hemostatic solutions can be used as hemostatic agents. For evaluation of blood coagulation, the first solution and second solution prepared in Example 1 and blood were mixed together at a ratio of 1:1:1 (each 200 µl), and the reaction between them was evaluated. The blood used contained the anticoagulant EDTA that prevents blood coagulation and platelet aggregation. The hemostatic solutions and blood were mixed together and allowed to react. After 30 seconds of the reaction, the blood coagulation performance of the hemostatic solutions was evaluated by observing the reaction rate of the hemostatic agents, the physical properties (visual) of the hydrogel, the amount of unreacted blood, and the uniformity of mixing with the blood. The results of the evaluation are shown in FIGS. 2, 3 and 4.

Comparative Examples 1 to 4 were performed in the same method as Example 3, except that solutions of pre-modified chitosan dissolved in 1% acetic acid, and dextran, carboxymethyl cellulose and hyaluronic acid solutions were prepared and the blood coagulation actions of combinations thereof were evaluated. The results of the evaluation are shown in FIG. 2. Specifically, FIG. 2 shows the results of blood coagulation evaluation of a combination of 2.5% chitosan (Mw: 50 to 190 kDa) solution and 8% dextran (40 kDa) solution in Comparative Example 1, a combination of 2.5% chitosan (Mw: 50 to 190 kDa) solution and 8% dextran (150 kDa) solution in Comparative Example 2, a combination of 2.5% chitosan (Mw: 50 to 190 kDa) solution and 10% carboxymethyl cellulose (90 kDa) solution in Comparative Example 3, and a combination of 2.5% chitosan (Mw: 50 to 190 kDa) solution and 1% hyaluronic acid (700 kDa) solution in Comparative Example 4. As a result, it was confirmed that no blood coagulation occurred.

Comparative Example 5-1 through Comparative Example 6-2 were performed in the same method as Example 3, except that solutions of chitosan introduced with functional groups different from those used in the present invention, and oxidized glucose or cellulose compound solutions were prepared and the blood coagulation actions of combinations thereof were evaluated. The results of the evaluation are shown in FIG. 3. Specifically, FIG. 3 shows the results of evaluating the blood coagulation action of a combination of 2.5% succinyl modified chitosan and 10% CMC-Al in Comparative Example 5-1, the blood coagulation action of a combination of 2.5% succinyl modified chitosan and DEX-Al in Comparative Example 5-2, the blood coagulation action of a combination of 2.5% trimethyl chitosan and 1% HA-Al in Comparative Example 6-1, and the blood coagulation action of a combination of 2.5% trimethyl chitosan and 10% CMC-Al in Comparative Example 6-2. As shown therein, non-uniform gels and unreacted materials were observed, and thus the solutions were not suitable as hemostatic agents. That is, after the functional group was introduced to the amino group of the side chain of chitosan, a problem arose in that the cationic property of the chitosan was lost and the inherent performance of the chitosan was degraded. Due to this problem, desired blood coagulation was not induced, even though the chitosan partially formed a gel.

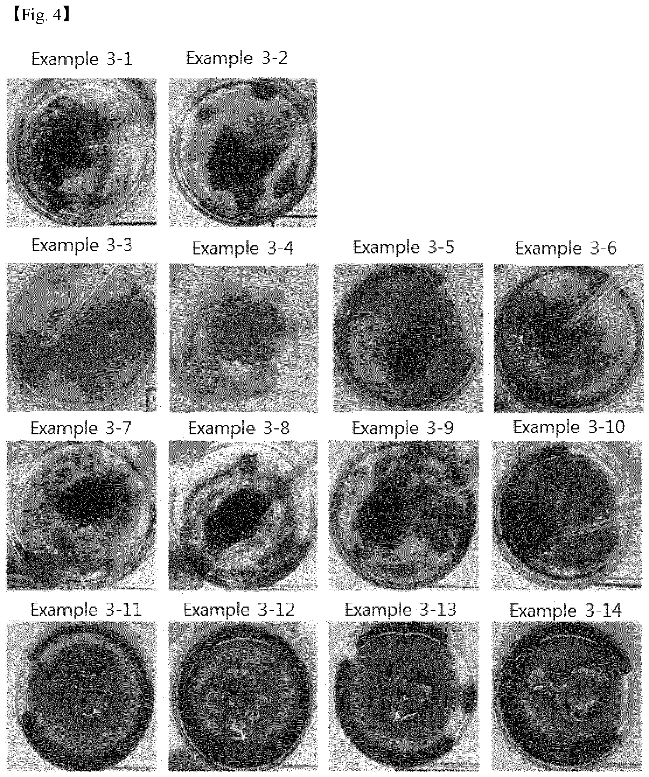

Examples 3-1 to 3-10 were performed in the same method as Example 3 to evaluate blood coagulation, except that CHI-Ly and DEX-Al solutions were used. Specifically, the blood coagulation actions of the following combinations of solutions were evaluated: a combination of 2.5% CHI-Ly (Mw: 50 to 190 kDa) solution and 20% DEX-Al (10 kDa) solution in Example 3-1; a combination of 1.25% CHI-Ly (Mw: 190 to 310 kDa) solution and 20% DEX-Al (10 kDa) solution in Example 3-2; a combination of 2.5% CHI-Ly (Mw: 50 to 190 kDa) solution and 8% DEX-Al (40 kDa) solution in Example 3-3; a combination of 1.25% CHI-Ly (Mw: 310 to 375 kDa) solution and 8% DEX-Al (40 kDa) solution in Example 3-4; a combination of 2.5% CHI-Ly (Mw: 50 to 190 kDa) solution and 8% DEX-Al (150 kDa) solution in Example 3-5; a combination of 1.25% CHI-Ly (Mw: 310 to 375 kDa) solution and 8% DEX-Al (150 kDa) solution in Example 3-6; a combination of 2.5% CHI-Ly (Mw: 50 to 190 kDa) solution and 9% DEX-Al (10 kDa) solution in Example 3-7; a combination of 1.25% CHI-Ly (Mw: 190 to 310 kDa) solution and 9% DEX-Al (10 kDa) solution in Example 3-8; a combination of 2.5% CHI-Ly (Mw: 50 to 190 kDa) solution and 8% DEX-Al (200 kDa) solution in Example 3-9; and a combination of 2.5% CHI-Ly (Mw: 190 to 310 kDa) solution and 8% DEX-Al (200 kDa) solution in Example 3-10. The results of the evaluation are shown in FIG. 4, and as shown therein, the blood coagulation performance did differ depending on the molecular weight and concentration of the material.

Examples 3-11 to 3-14 were performed in the same method as Example 3 to evaluate blood coagulation, except that CHI-Ly solution and HA-Al or CMC-Al solution were used in combination. Specifically, the blood coagulation actions of the following combinations of solutions were evaluated: 2.5% CHI-Ly (Mw: 50 to 190 kDa) solution and 2% HA-Al (3,000 kDa) solution in Example 3-11; 1.25% CHI-Ly (Mw: 190 to 310 kDa) solution and 2% HA-Al (3,000 kDa) solution in Example 3-12; 2.5% CHI-Ly (Mw: 50 to 190 kDa) and 10% CMC-Al (90 kDa) solution in Example 3-13; and 1.25% CHI-Ly (Mw: 190 to 310 kDa) solution and 10% CMC-Al (90 kDa) solution in Example 3-14. The results of the evaluation are shown in FIG. 4, and as shown therein, the hydrogel was formed within a short time, but a certain amount of unreacted blood was observed.

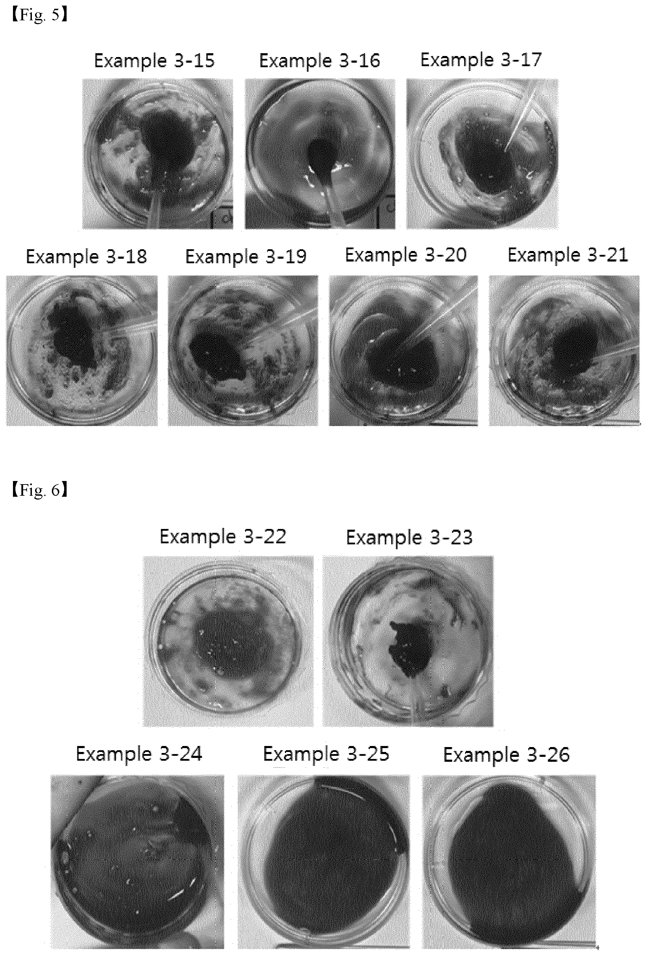

Examples 3-15 to 3-17 were performed in the same method as Example 3 to evaluate blood coagulation, except that CHI-Sh solution and DEX-Al, HA-Al or ALG-Sh solution were used in combination. Specifically, the blood coagulation actions of the following combinations of solutions were evaluated: 2.5% CHI-Sh (Mw: 190 to 310 kDa) solution and 8% DEX-Al (150 kDa) solution in Example 3-15; 2.5% CHI-Sh (Mw: 190 to 310 kDa) solution and 2% HA-Al (700 kDa) solution in Example 3-16; and 2.5% CHI-Sh (Mw: 190 to 310 kDa) solution and 10% ALG-Sh (120 to 190 kDa) solution in Example 3-17. The results of the evaluation are shown in FIG. 5, and as shown therein, it was observed that blood coagulation occurred within a short time, and the degree of blood coagulation did differ depending on the kind of oxidized glucose.

Examples 3-18 to 3-21 were performed in the same method as Example 3 to evaluate blood coagulation, except that CHI-Sh solution and DEX-Al solution were used. Specifically, the blood coagulation actions of the following combinations of solutions were evaluated: 2.5% CHI-Sh (Mw: 50 to 190 kDa) solution and 8% DEX-Al (40 kDa) solution in Example 3-18; 2.5% CHI-Sh solution (Mw: 3 to 20 kDa) solution and 8% DEX-Al (40 kDa) solution in Example 3-19; 2.5% CHI-Sh (Mw: 50 to 190 kDa) solution and 9% DEX-Al (40 kDa) solution in Example 3-20; and 2.5% CHI-Sh (Mw: 3 to 20 kDa) solution and 9% DEX-Al (40 kDa) solution in Example 3-21. The results of the evaluation are shown in FIG. 5, and it could be seen that blood coagulation occurred within a short time.

Example 3-22 was performed in the same method as Example 3 to evaluate blood coagulation, except that CHI-Ar solution and DEX-Al or CMC-Al solution were used in combination. Specifically, the blood coagulation action of a combination of 2.5% CHI-Ar (Mw: 50 to 190 kDa) solution and 8% DEX-Al (40 kDa) solution was evaluated, and the result is shown in FIG. 6.

Examples 3-23 and 3-24 were performed in the same method as Example 3 to evaluate blood coagulation, except that representative cationic polymer polyethyleneimine (PEI) solution and DEX-Al or CMC-Al solution were used in combination. Specifically, the blood coagulation actions of the following combinations of solutions were evaluated: 25% PEI (Mw: 20 kDa) solution and 10% CMC-Al (9 kDa) solution in Example 3-23; and 25% PEI (Mw: 20 kDa) solution and 8% DEX-Al (Mw: 40 kDa) solution in Example 3-24. The results of the evaluation are shown in FIG. 6, and it can be seen that the blood coagulation performance did significantly differ depending on the kind of second component.

Examples 3-25 and 3-26 were performed in the same method as Example 3 to evaluate blood coagulation, except that CHI-Ly solution and ALG-Al solution were used in combination. Specifically, the blood coagulation actions of the following combinations of solutions were evaluated: 2.5% CHI-Ly (Mw: 50 to 190 kDa) solution and 10% ALG-Al (Mw: 120 to 190 kDa) solution in Example 3-25; and 1.25% CHI-Ly (Mw: 190 to 310 kDa) solution and 10% ALG-Al (Mw: 120 to 190 kDa) solution in Example 3-26. The results of the evaluation are shown in FIG. 6, and as shown therein, the solutions partially showed blood coagulation, but a slight amount of unreacted blood was observed.

### <Example 4> Evaluation of Physical Properties of Hydrogel

The physical properties of hemostatic solutions were quantitatively evaluated by measuring the viscosity, storage modulus (G') and loss modulus (G") of the first solution and second solution prepared in Example 1.

In addition, in order to measure the physical properties of the blood clot generated by blood coagulation with the hemostatic solutions, the first solution, the second solution, and blood were allowed to react at a ratio of 1:1:1, and the physical properties were measured.

As a rheometer, an Anton-Paar (MCR 102) rheometer was used. The hemostatic solutions and blood were loaded onto a 25-mm-diameter plate, and measurement was performed under the following set conditions: a plate gap of 0.5 mm, a frequency sweep of 0.1 to 10 Hz, an oscillating frequency strain of 1%, and a measurement temperature of 37°C.

Measurement of physical properties was carried out by selecting the material combinations forming the blood clot from the above-described Examples, and quantitative evaluation was performed through the storage modulus and viscosity values at frequency 1. Specifically, the measured storage moduli were 5.7*10³ Pa for Example 3-1, 2.0*10³ Pa for Example 3-3, 1.6*10³ Pa for Example 3-5, 8.3*10² Pa for Example 3-9, 6.3*10² Pa for Example 3-15, 1.5*10² Pa for Example 3-16, 1.2*10² Pa for Example 3-17, and 2.0*10³ Pa for Example 3-23. In addition, the measured viscosities were 9.0*10² Pa·s for Example 3-1, 3.2*10² Pa·s for Example 3-3, 2.5*10² Pa·s for Example 3-5, 1.3*10² Pa·s for Example 3-9, 1.0*10² Pa·s for Example 3-15, 2.0*10¹ Pa·s for Example 3-16, 7.8*10¹ Pa·s for Example 3-17, and 3.2*10² Pa·s for Example 3-23.

Referring to the results in FIG. 7, when the ratio of the oxidizing agent per repeating unit of the second compound having an aldehyde group introduced therein by treatment with the oxidizing agent periodate increased, the degree of oxidation and the physical properties increased. Specifically, the measured storage moduli were 9.2*10² Pa at 0.5 equivalents of the oxidizing agent per repeating unit, 1.6*10³ Pa at 1 equivalent of the oxidizing agent per repeating unit, 1.7*10³ Pa at 1.5 equivalents of the oxidizing agent per repeating unit, and 1.7*10³ Pa at 2 equivalents of the oxidizing agent per repeating unit. Thus, treatment with 1.5 equivalents or more of periodate per repeating unit is preferable.

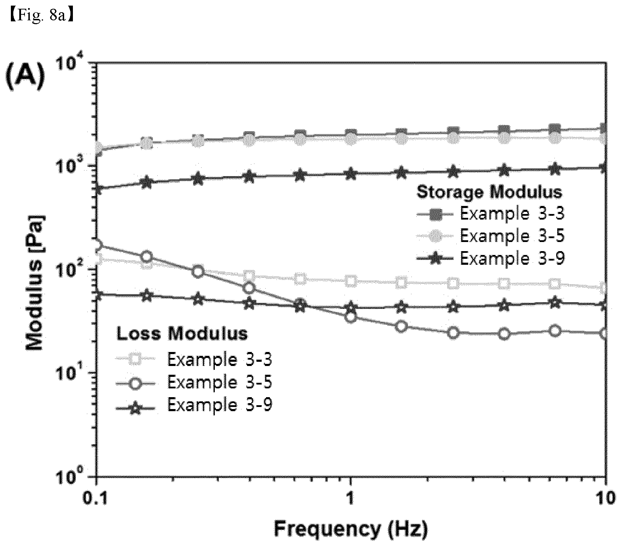

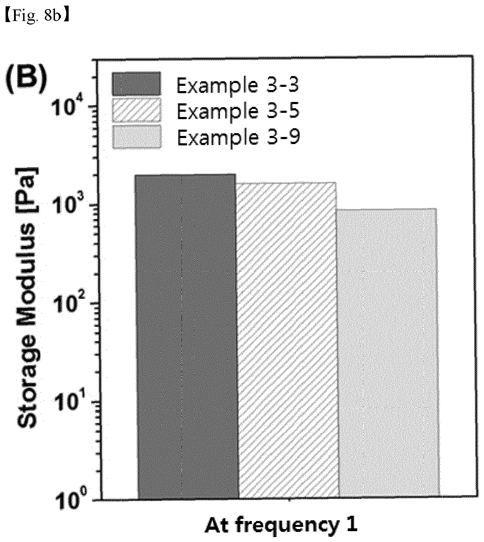

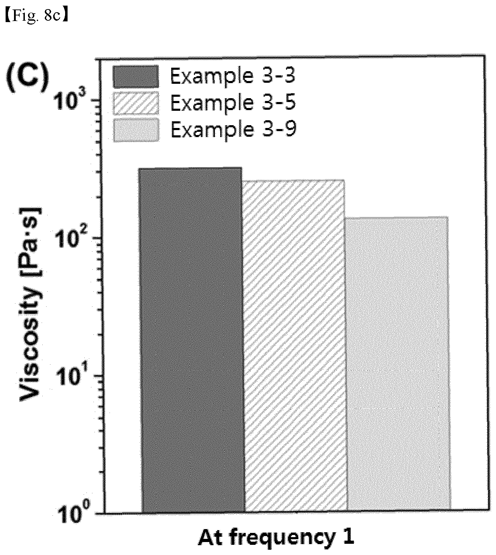

Referring to the results in FIG. 8, when other parameters of the hemostatic solutions were fixed and the physical properties of the blood clot were measured as a function of the molecular weight of oxidized dextran (Examples 3-3, 3-5 and 3-9), the physical properties were higher as the molecular weight was lower, and the same results were shown for chitosan as the first component.

### <Example 5> Comparative Evaluation of Liquid Hemostatic Agent (Tisseel) and Hydrogel-Type Hemostatic Agent

Comparative evaluation was made by performing a blood coagulation test for the commercially available liquid hemostatic agent (Tisseel, Baxter) in the same method as in Example 3. Tisseel is a two-liquid type hemostatic agent containing blood products and having effects such as local hemostasis, suture, and tissue adhesion. The first solution (fibrin sealer) is composed of additives including fibrinogen, aprotinin and human albumin, and the second solution (thrombin solution) is composed of additives including human thrombin, calcium chloride dihydrate and human albumin. The first solution, the second solution, and blood were mixed at a ratio of 1:1:1 (each 100 µl) and allowed to react. After 30 seconds of the reaction, the reaction rate, the physical properties (visual) of the hydrogel, the amount of unreacted blood, and the uniformity of mixing with the blood were evaluated.

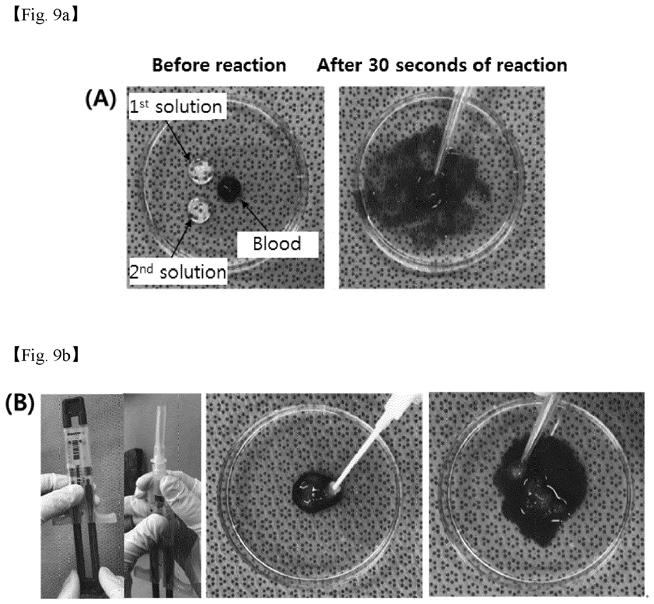

The results of the evaluation are shown in FIG. 9(A), and as shown therein, the reaction occurred within a few seconds, but the blood and the components were not uniformly mixed, and the reaction between the first solution and the second solution occurred first, not blended with blood, and thus unreacted blood was observed.

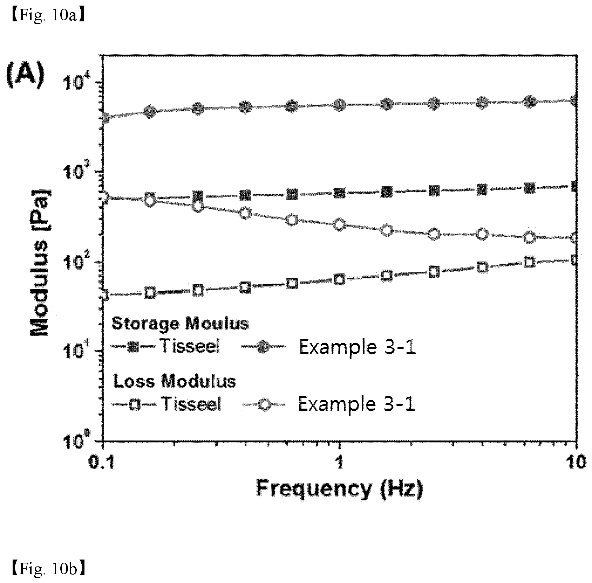

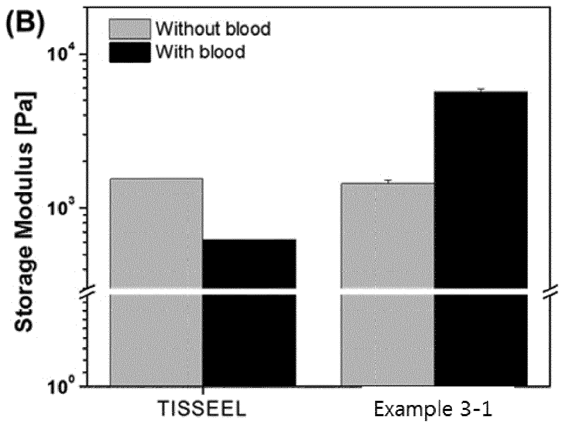

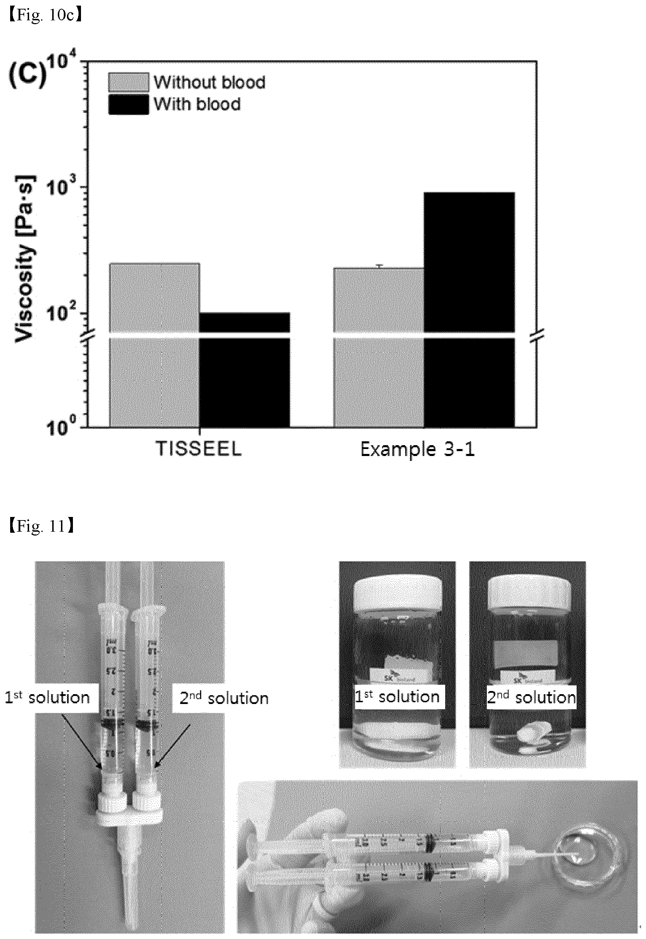

In addition, in FIG. 9(B), similar results were observed in evaluation performed using a dual chamber syringe and an application cannula. The physical properties of the blood clot formed by mixing Tisseel with blood were evaluated using a rheometer in the same manner as in Example 4. The results of comparison with Example 3-1 are shown in FIG. 10, and as shown therein, the components of the Examples exhibit relatively high physical properties. Specifically, the storage modulus and viscosity of Tisseel at frequency 1 were measured to be 6.2*10² Pa and 1.0*10² Pa·s, respectively. In addition, it was confirmed that the physical properties of Tisseel decreased after mixing with blood and blood coagulation, whereas the physical properties of the components of Example 3-1 increased, suggesting that the components of Example 3-1 induce blood coagulation by reaction with blood components.

FIG. 11 shows that the first solution and second solution of the present invention are transparent liquids having fluidity before mixing, and may also be applied in a mixed state by means of a dual chamber syringe as needed.

Although the present invention has been described with reference to the above embodiments, these embodiments are merely exemplary. Therefore, the true technical protection scope of the present invention will be defined by the technical details of the appended claims.

### Industrial Applicability

As described above, according to the present invention, the hydrogel-type hemostatic agent is a hemostatic agent that does not contain blood products such as thrombin and fibrinogen, and may be applied to hemophilia patients and diabetic patients who have impairment in the blood coagulation system, and patients taking an anticoagulant and aspirin, in order to induce blood coagulation and hemostasis. In addition, it may be used as a hemostatic agent having a more effective hemostatic effect in a wide range of surgical operation areas because multiple reactions occur simultaneously.

## Claims

1. A two-liquid type composition for use as a blood coagulation agent, said composition comprising:
a first solution containing the first component which is a chitosan compound having a cationic amino acid conjugated to a side chain thereof; and
a second solution containing the second component which is selected from dextran, hyaluronic acid, carboxymethyl cellulose or alginate compound having an aldehyde group introduced therein by oxidation,
wherein, when the first solution and the second solution are mixed together, a hydrogel is formed by a Schiff base reaction between an amine (-NH₂) functional group of the chitosan compound and an aldehyde (-CHO) functional group of the second component simultaneously with electrostatic interaction between opposite charges of the chitosan compound and the second component.

2. The two-liquid type composition for use according to claim 1, wherein, when the first solution and the second solution are applied to a bleeding site, the following steps occur simultaneously:
a step of inducing blood coagulation by electrostatic interaction between opposite charges of material and material and between opposite charges of material and blood;
a step of inducing blood coagulation by a Schiff base reaction between material and material and between material and blood; and
a step of stopping bleeding by physically pressing a bleeding site by a hydrogel formed through sol-gel transition that occur through multiple reactions.

3. The two-liquid type composition for use according to claim 1, wherein the chitosan compound of the first solution independently has a cationic property, is a chitosan compound in which one or more cationic amino acids selected from among arginine, histidine, cysteine and lysine, which contain one carboxyl group and one or more amino groups, are conjugated to a side chain of the chitosan compound, and the chitosan compound dissolves at neutral pH.

4. A method for preparing the two-liquid type composition recited in claim 1, the method comprising steps of:
preparing a first solution by dissolving, in distilled water or physiological saline, the first component which is a chitosan compound having a cationic amino acid conjugated to a side chain thereof; and
preparing a second solution by dissolving, in distilled water or physiological saline, the second component which is selected from dextran, hyaluronic acid, carboxymethyl cellulose or alginate compound having an aldehyde group introduced therein by oxidation.

5. The two-liquid type composition for use according to claim 1, wherein the first solution and the second solution are stored in separate chambers, and when the first solution and the second solution are applied to a bleeding site, they are mixed together, thus immediately forming a hydrogel in-situ at the bleeding site.

6. The two-liquid type composition for use according to claim 5, wherein the first solution and the second solution are contained in a dual chamber syringe, and a method of applying the first and second solutions is selected by assembling one of a needle, a mixing nozzle, a spreader and a spray-type type auxiliary tool to an inlet of the dual chamber syringe.

7. The two-liquid type composition for use according to claim 1, wherein any one or both of the first solution and the second solution further contain cells, a drug or an anti-adhesion material.

8. The two-liquid type composition for use according to claim 7, wherein the two-liquid type composition comprises the cells and functions as a tissue engineering scaffold by the formed hydrogel.

9. The two-liquid type composition for use according to claim 7, wherein the two-liquid type composition comprises the drug and functions as a drug carrier by the formed hydrogel.

10. The two-liquid type composition for use according to claim 7, wherein the two-liquid type composition comprises the anti-adhesion material and functions as an anti-adhesion agent by the formed hydrogel.

## Patentansprüche

1. Zusammensetzung des Zweiflüssigkeitstyps für die Verwendung als ein Blutgerinnungsmittel, wobei die Zusammensetzung umfasst:
eine erste Lösung, die die erste Komponente enthält, die eine Chitosanverbindung mit einer kationischen Aminosäure ist, die an eine Seitenkette davon konjugiert ist, und
eine zweite Lösung, die die zweite Komponente enthält, die aus Dextran, Hyaluronsäure, Carboxymethylcellulose oder einer Alginatverbindung mit einer durch Oxidation darin eingeführten Aldehydgruppe ausgewählt ist,
wobei, wenn die erste Lösung und die zweite Lösung miteinander vermischt werden, ein Hydrogel durch eine Schiff-Base-Reaktion zwischen einer funktionellen Amin- (-NH₂) Gruppe der Chitosanverbindung und einer funktionellen Aldehyd- (-CHO) Gruppe der zweiten Komponente gleichzeitig mit einer elektrostatischen Wechselwirkung zwischen entgegengesetzten Ladungen der Chitosanverbindung und der zweiten Komponente ausgebildet wird.

2. Zusammensetzung des Zweiflüssigkeitstyps für die Verwendung nach Anspruch 1, wobei, wenn die erste Lösung und die zweite Lösung auf eine blutende Stelle aufgebracht werden, die folgenden Schritte gleichzeitig stattfinden:
ein Schritt des Induzierens der Blutgerinnung durch elektrostatische Wechselwirkung zwischen entgegengesetzten Ladungen von Material und Material und zwischen entgegengesetzten Ladungen von Material und Blut;
ein Schritt des Induzierens der Blutgerinnung durch eine Schiff-Base-Reaktion zwischen Material und Material und zwischen Material und Blut; und
einen Schritt des Stoppens von Blutungen durch physikalisches Pressen einer blutenden Stelle durch ein Hydrogel, das durch Sol-Gel-Übergang ausgebildet wird, der durch mehrere Reaktionen auftritt.

3. Zusammensetzung des Zweiflüssigkeitstyps für die Verwendung nach Anspruch 1, wobei die Chitosanverbindung der ersten Lösung unabhängig eine kationische Eigenschaft aufweist, eine Chitosanverbindung ist, in der eine oder mehrere kationische Aminosäuren, ausgewählt aus Arginin, Histidin, Cystein und Lysin, die eine Carboxylgruppe und eine oder mehrere Aminogruppen enthalten, an eine Seitenkette der Chitosanverbindung konjugiert sind, und die Chitosanverbindung sich bei neutralem pH-Wert auflöst.

4. Verfahren zum Herstellen der Zusammensetzung des Zweiflüssigkeitstyps nach Anspruch 1, wobei das Verfahren die Schritte umfasst:
Herstellen einer ersten Lösung durch Auflösen der ersten Komponente, die eine Chitosanverbindung mit einer an eine Seitenkette davon konjugierten kationischen Aminosäure ist, in destilliertem Wasser oder physiologischer Kochsalzlösung, und
Herstellen einer zweiten Lösung durch Auflösen der zweiten Komponente, die aus Dextran, Hyaluronsäure, Carboxymethylcellulose oder einer Alginatverbindung mit einer durch Oxidation darin eingeführten Aldehydgruppe ausgewählt ist, in destilliertem Wasser oder physiologischer Kochsalzlösung,

5. Zusammensetzung des Zweiflüssigkeitstyps für die Verwendung nach Anspruch 1, wobei die erste Lösung und die zweite Lösung in getrennten Kammern gelagert werden und, wenn die erste Lösung und die zweite Lösung auf eine blutende Stelle aufgebracht werden, sie miteinander vermischt werden, wodurch sofort ein Hydrogel *in-situ* an der blutenden Stelle ausgebildet wird.

6. Zusammensetzung des Zweiflüssigkeitstyps für die Verwendung nach Anspruch 5, wobei die erste Lösung und die zweite Lösung in einer Zweikammerspritze enthalten sind und ein Verfahren zum Auftragen der ersten und der zweiten Lösung durch Zusammenbauen einer/eines einer Nadel, einer Mischdüse, eines Verteilers und eines Hilfswerkzeugs des Sprühtyps an einem Einlass der Zweikammerspritze ausgewählt wird.

7. Zusammensetzung des Zweiflüssigkeitstyps für die Verwendung nach Anspruch 1, wobei eine oder beide der ersten Lösung und der zweiten Lösung ferner Zellen, ein Arzneimittel oder ein Antiadhäsionsmaterial enthalten.

8. Zusammensetzung des Zweiflüssigkeitstyps für die Verwendung nach Anspruch 7, wobei die Zusammensetzung des Zweiflüssigkeitstyps die Zellen umfasst und durch das ausgebildete Hydrogel als ein Gerüst für die Gewebezüchtung wirkt.

9. Zusammensetzung des Zweiflüssigkeitstyps für die Verwendung nach Anspruch 7, wobei die Zusammensetzung des Zweiflüssigkeitstyps das Arzneimittel umfasst und durch das ausgebildete Hydrogel als ein Arzneimittelträger wirkt.

10. Zusammensetzung des Zweiflüssigkeitstyps für die Verwendung nach Anspruch 7, wobei die Zusammensetzung des Zweiflüssigkeitstyps das Antiadhäsionsmaterial umfasst und durch das ausgebildete Hydrogel als ein Antiadhäsionsmittel wirkt.

## Revendications

1. Composition de type à deux liquides à utiliser comme agent de coagulation sanguine, ladite composition comprenant :
une première solution contenant le premier composant qui est un composé de chitosane ayant un acide aminé cationique conjugué à une chaîne latérale de celui-ci ; et
une seconde solution contenant le second composant qui est choisi parmi le dextrane, l'acide hyaluronique, la carboxyméthylcellulose ou un composé alginate dans lequel un groupe aldéhyde est introduit par oxydation,
dans laquelle, lorsque la première solution et la seconde solution sont mélangées ensemble, un hydrogel est formé par une réaction de base de Schiff entre un groupe fonctionnel amine (-NH₂) du composé chitosane et un groupe fonctionnel aldéhyde (-CHO) du second composant simultanément à une interaction électrostatique entre les charges opposées du composé chitosane et le second composant.

2. Composition de type à deux liquides à utiliser selon la revendication 1, dans laquelle, lorsque la première solution et la seconde solution sont appliquées sur un site de saignement, les étapes suivantes se produisent simultanément :
une étape consistant à induire une coagulation sanguine par interaction électrostatique entre des charges opposées d'un matériau et d'un matériau et entre des charges opposées d'un matériau et de sang ;
une étape consistant à induire une coagulation sanguine par une réaction de base de Schiff entre un matériau et un matériau et entre un matériau et du sang ; et
une étape consistant à arrêter le saignement en appuyant physiquement sur un site de saignement par un hydrogel formé par une transition sol-gel qui se produit par de multiples réactions.

3. Composition de type à deux liquides à utiliser selon la revendication 1, dans laquelle le composé chitosane de la première solution possède indépendamment une propriété cationique, est un composé chitosane dans lequel un ou plusieurs acides aminés cationiques choisis parmi l'arginine, l'histidine, la cystéine et la lysine, qui contiennent un groupe carboxyle et un ou plusieurs groupes amino, sont conjugués à une chaîne latérale du composé chitosane, et le composé chitosane se dissout à pH neutre.

4. Procédé de préparation de la composition de type à deux liquides selon la revendication 1, le procédé comprenant les étapes suivantes :
la préparation d'une première solution en dissolvant, dans de l'eau distillée ou du sérum physiologique, le premier composant qui est un composé de chitosane ayant un acide aminé cationique conjugué à une chaîne latérale de celui-ci ; et
la préparation d'une seconde solution en dissolvant, dans de l'eau distillée ou du sérum physiologique, le second composant qui est choisi parmi le dextrane, l'acide hyaluronique, la carboxyméthylcellulose ou un composé alginate dans lequel un groupe aldéhyde est introduit par oxydation.

5. Composition de type à deux liquides à utiliser selon la revendication 1, dans laquelle la première solution et la seconde solution sont stockées dans des chambres séparées, et lorsque la première solution et la seconde solution sont appliquées sur un site de saignement, elles sont mélangées ensemble, donc immédiatement formant un hydrogel in situ au site de saignement.

6. Composition de type à deux liquides à utiliser selon la revendication 5, dans laquelle la première solution et la seconde solution sont contenues dans une seringue à double chambre, et un procédé d'application des première et seconde solutions est sélectionné en assemblant l'un d'une aiguille, d'un mélangeur buse, d'un épandeur et un d'outil auxiliaire de type pulvérisateur à une entrée de la seringue à double chambre.

7. Composition de type à deux liquides à utiliser selon la revendication 1, dans laquelle l'une ou les deux de la première solution et de la seconde solution contiennent en outre des cellules, un médicament ou un matériau anti-adhérent.

8. Composition de type à deux liquides à utiliser selon la revendication 7, dans laquelle la composition de type à deux liquides comprend les cellules et fonctionne comme un échafaudage d'ingénierie tissulaire grâce à l'hydrogel formé.

9. Composition de type à deux liquides à utiliser selon la revendication 7, dans laquelle la composition de type à deux liquides comprend le médicament et fonctionne comme support de médicament par l'hydrogel formé.

10. Composition de type à deux liquides à utiliser selon la revendication 7, dans laquelle la composition de type à deux liquides comprend le matériau anti-adhérent et fonctionne comme un agent anti-adhérent par l'hydrogel formé.
